# EUROPÄISCHE PATENTSCHRIFT

(11) **EP 0 991 781 B1**
(45) Veröffentlichungstag und Bekanntmachung des Hinweises auf die Patenterteilung: **29.11.2006**
(21) Anmeldenummer: 98954248.5
(22) Anmeldetag: 12.08.1998
(51) Int. Cl.: C12Q 1/68

(54) **NUKLEINSÄUREMOLEKÜLSATZ FÜR SALMONELLA-NACHWEIS, NUKLEINSÄUREN, KIT UND VERWENDUNG**
GROUP OF NUCLEIC ACID MOLECULES FOR SALMONELLA DETECTION, NUCLEIC ACIDS, KIT AND USE
GROUPE DE MOLECULES D'ACIDE NUCLEIQUE POUR LA CARACTERISATION DE LA SALMONELLE, ACIDES NUCLEIQUES, NECESSAIRE D'ESSAI ET UTILISATION

(30) Priorität: 12.08.1997 DE 19734940
(43) Veröffentlichungstag der Anmeldung: 12.04.2000
(73) Patentinhaber: Biotecon Diagnostics GmbH, 10589 Berlin (DE)
(72) Erfinder: BERGHOF, Cornelia, D-12355 Berlin (DE); GASCH, Alexander, D-13359 Berlin (DE); SCHEU, Pia, D-12101 Berlin (DE); WILBORN, Freimut, D-14057 Berlin (DE)
(74) Vertreter: Boeters, Hans Dietrich
(86) Internationale Anmeldenummer: PCT/EP1998/005129
(87) Internationale Veröffentlichungsnummer: WO 1999/007886

(56) Entgegenhaltungen:
- EP-A- 0 314 294
- EP-A- 0 335 633
- EP-A- 0 479 117
- EP-A- 0 721 989
- WO-A-95/00664

### SEQUENZPROTOKOLL

(1) ALLGEMEINE ANGABEN:
   (i) ANMELDER:
      (A) NAME: Biotecon Gesellschaft fuer Biotechnologische Entwicklung und Consulting mbH
      B) STRASSE: Gustav-Meyer-Allee 25
      (C) ORT: Berlin
      (E) LAND: Deutschland
      (F) POSTLEITZAHL: 13355

      (A) NAME: Cornelia Berghof
      (B) STRASSE: Rhodelaenderweg 85
      (C) ORT: Berlin
      (E) LAND: Deutschland
      (F) POSTLEITZAHL: 12355

      (A) NAME: Alexander Gasch
      (B) STRASSE: Steegerstr. 71
      (C) ORT: Berlin
      (E) LAND: Deutschland
      (F) POSTLEITZAHL: 13359

      (A) NAME: Pia scheu
      (B) STRASSE: Boelckestr. 45
      (C) ORT: Berlin
      (E) LAND: Deutschland
      (F) POSTLEITZAHL: 12101

      (A) NAME: Freimut Wilborn
      (B) STRASSE: Neue Kantstr. 9
      (C) ORT: Berlin
      (E) LAND: Deutschland
      (F) POSTLEITZAHL: 14057
   (ii) BEZEICHNUNG DER ERFINDUNG: Nucleinsaeuremolekuel-Satz fuer salmonella-Nachweis, Nucleinsaeuren, Kit und Verwendung
   (iii) ANZAHL DER SEQUENZEN: 13
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (v) DATEN DER JETZIGER ANMELDUNG: ANMELDENUMMER: WO 98/05129
(2) ANGABEN ZU SEQ ID NO: 1:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iV) ANTISENSE: NEIN
   (xi) SEQUENZBESCHREIBUNG: SEQ ID NO: 1:
      ATGGATCAGA ATACGCCCCG 20
(2) ANGABEN ZU SEQ ID NO: 2:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 2:
      ATGGATCAGA ATACACCCCG 20
(2) ANGABEN ZU SEQ ID NO: 3:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 3:
      CAGAATACGC CCCGTTCGGC 20
(2) ANGABEN ZU SEQ ID NO: 4:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 4:
      CAGAATACAC CCCGTTCGGC 20
(2) ANGABEN ZU SEQ ID NO: 5:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 5:
      CAGAATACGC CCCGTTCAGC 20
(2) ANGABEN ZU SEQ ID NO: 6:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (iii) HYPOTHETISCH: NEIN
   (iV) ANTISENSE: NEIN
   (xi) SEQUENZBESCHREIBUNG: SEQ ID NO: 6:
      CAACCTAACT TCTGCGCCAG 20
(2) ANGABEN ZU SEQ ID NO: 7:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 7:
      CAACCTAACT TCTGCACCAG 20
(2) ANGABEN ZU SEQ ID NO: 8:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 8:
      CAACCTAACC TCTGCGCCAG 20
(2) ANGABEN ZU SEQ ID NO: 9:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 9:
      CAACCTAACT TCTGCGCCAG 20
(2) ANGABEN ZU SEQ ID NO: 10:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 10:
      CAGCCTAACT TCTGCGCCAG 20
(2) ANGABEN ZU SEQ ID NO: 11:
   (i) SEQUENZKENNZEICHEN:
      (A) LÄNGE: 25 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 11:
      AGCCAACCAT TGCTAAATTG GCGCA 25
(2) ANGABEN ZU SEQ ID NO: 12:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 12:
      GGTAGAAATT CCCAGCGGGT ACTG 24
(2) ANGABEN ZU SEQ ID NO: 13:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
      (A) BESCHREIBUNG: /desc = "Primer"
   (iii) HYPOTHETISCH: NEIN
   (iv) ANTISENSE: NEIN
   (xi) SEQUENZBESCHREIBUNG: SEQ ID NO: 13:
      TTTGCGACTA TCAGGTTACC GTGG 24

## Beschreibung

Die Kontamination von Produkten der Lebensmittel- und Pharmaindustrie über die eingesetzten Rohstoffe oder während des Produktions- oder Verpackungsprozesses durch pathogene Mikroorganismen stellt ein großes Problem dar. Salmonellen gehören zu den wichtigsten durch Lebensmittel übertragenen Krankheitserregern des Menschen. Da die Detektion und Identifikation von Salmonellen mit konventionellen mikrobiologischen Nachweisverfahren sehr zeitaufwendig ist - für die in gesetzlichen Vorschriften (LMBG, FDA) geforderte Anreicherung und nachfolgende Serotypisierung werden mindestens fünf Tage benötigt - besteht ein großer Bedarf nach alternativen Schnellmethoden.

Für den routinemäßigen Einsatz zur Erfassung von Mikroorganismen sind in den letzten Jahren eine Reihe neuer Methoden entwickelt worden. Hierzu zählen immunologische Verfahren, die auf dem Einsatz polyvalenter oder monoklonaler Antikörper beruhen und Verfahren, bei denen Nucleinsäure-Sonden zum Nachweis mittels Hybridisierung an keimspezifische Nucleinsäuren eingesetzt werden. Als weitere Methoden werden diejenigen Verfahren beschrieben, die auf einer spezifischen Nucleinsäure-Amplifikation basieren, mit oder ohne anschließende Bestätigungsreaktion durch NucleinsäureHybridisierung. Geeignete Verfahren zur Amplifikation von Nucleinsäuren sind z.B. die Polymerase-Kettenreaktion (polymerase chain reaction, PCR) [US Patente 4,683,195; 4,683,202; und 4,965,188], die Ligase-Kettenreaktion [WO Veröffentlichung 89/09835], die "self-sustained sequence replication" [EP 329,822], das "transcription based amplification system" [EP 310,229] und das Qβ RNA-Replikase-System [US Patent 4,957,858].

Die genannten auf Nucleinsäuren basierenden Verfahren sind so sensitiv, daß, anders als bei konventionellen mikrobiologischen Verfahren, eine langwierige Anreicherung des nachzuweisenden Mikroorganismus aus der zu untersuchenden Probe entfällt. Eine Untersuchung auf An- oder Abwesenheit von z.B. Salmonellen ist daher bei Anwendung der genannten auf Nucleinsäuren basierenden Verfahren in der Regel innerhalb eines Arbeitstages abgeschlossen.

Einige Nucleinsäuresequenzen zum Nachweis von Salmonellen mit der Polymerase-Kettenreaktion sind bekannt. Nachteilig ist jedoch, daß bei Einsatz dieser Nucleinsäuresequenzen als Primer bei der Polymerase-Kettenreaktion falsch positive [WO 95/33854] oder falsch negative [WO 92/01056; WO 95/00664; WO 92/01056; WO 93/04202] Resultate auftreten. In anderen Fällen sind nur eine unzureichende Anzahl von Stämmen aller 7 *Salmonella-*Subspezies untersucht [WO 92/08805; WO 94/25597; DE 4337295], so daß bisher unklar ist, ob die jeweiligen Nucleinsäuresequenzen zum Nachweis aller *Salmonella*-Stämme geeignet sind.

Vorteilhaft an z.B. den in der internationalen Patentanmeldung WO 95/00664 beschriebenen Primern und Sonden ist, daß sie den hochselektiven Nachweis von Bakterien der Gattung *Salmonella* erlauben, ohne daß falsch positive Resultate auftreten. Nachteilig beim Einsatz der Oligonucleotide gemäß WO 95/00664 in Amplifikationsverfahren wie der Polymerase-Kettenreaktion ist jedoch die Tatsache, daß mit keinem der beschriebenen Primerpaare alle Vertreter der 7 *Salmonella*-Subspezies erfaßt werden. So werden beispielsweise beim Einsatz der Primer ST11/ST15 mehrere Vertreter der Subspezies IIIa (subsp. *arizonae*), beim Einsatz der Primer ST11/ST14 mehrere Vertreter der Subspezies I (subsp. *enterica* Serovar. Blockley) und der Subspezies IIIa (subsp. *arizonae*) nicht detektiert.

Ziel der hier dargestellten Erfindung war die Optimierung der in WO 95/00664 beschriebenen Nachweisverfahren durch Ermittlung von Nucleinsäuresequenzen, deren Einsatz als Primer und/oder Sonden eine möglichst vollständige Erfassung aller Vertreter der Gattung *Salmonella* sicherstellt.

Gemäß einer Ausführungsform wird die der Erfindung zugrundeliegende Aufgabe durch einen Satz von Nucleinsäuremolekülen gelöst, mit dem sich bei einem Verfahren zum Nachweis von Vertretern von *Salmonella enterica* subsp. enterica, *salamae, arizonae, diarizonae, houtenae, bongori* und *indica* sämtliche Vertreter dieser Subspezies nachweisen lassen, wobei der Satz dadurch gewinnbar ist, daß man
(a) in an sich bekannter Weise mit Hilfe eines Nucleinsäure-Isolats eines Vertreters einer der genannten *Salmonella enterica*-Subspezies ein erstes Nucleinsäuremolekül (Nucleinsäuremolekül 1) gewinnt oder ableitet, das sich spezifisch als Primer oder Sonde zum Nachweis dieses Vertreters oder weiterer oder aller Vertreter dieser einen *Salmonella enterica*-Subspezies und gegebenenfalls zusätzlich von Vertretern weiterer *Salmonella enterica-*Subspezies eignet,
(b) in an sich bekannter Weise mit Hilfe eines Nucleinsäure-Isolats eines anderen Vertreters einer der genannten *Salmonella enterica-*Subspezies ein zweites Nucleinsäuremolekül (Nucleinsäuremolekül 2) gewinnt oder ableitet, das sich spezifisch als Primer oder Sonde zum Nachweis dieses Vertreters oder weiterer oder aller Vertreter dieser anderen *Salmonella enterica*-Subspezies und gegebenenfalls zusätzlich von Vertretern von weiteren der genannten *Salmonella enterica-*Subspezies eignet und
(c) sofern sich mit den gemäß (a) und (b) gewinnbaren Nucleinsäuremolekülen nicht bereits alle Vertreter der genannten *Salmonella enterica*-Subspezies nachweisen lassen, die Gewinnung oder Ableitung von Nucleinsäuremolekülen gemäß (a) und/oder (b) so lange fortsetzt, bis sich mit dem gewonnenen oder abgeleiteten Satz von Nucleinsäuremolekülen alle Vertreter der genannten *Salmonella enterica*-Subspezies nachweisen lassen.

Bei einem abgeleiteten Nucleinsäuremolekül kann es sich um ein Nucleinsäuremolekül handeln, das mit dem gewonnenen Nucleinsäuremolekül hybridisierbar ist und vorzugsweise dieselbe Basenanzahl aufweist, wobei Hybridisierungsbedingungen sein können:
Temperatur ≥ 25 °C und 1 M NaCl-Konzentration.
Bei einem abgeleiteten Nucleinsäuremolekül kann es sich beispielsweise um ein Nucleinsäuremolekül handeln, dessen Sequenz mittels Computerdesigning bestimmt worden ist und das nachfolgend durch chemische Synthese hergestellt und gewonnen worden ist.

Man kann die Lösung der der Erfindung zugrundeliegenden Aufgabe auch dahingehend beschreiben, daß ein oder mehrere Nucleinsäuremolekül(e) Y (Z, ... ) vorgesehen werden, wobei diese(s) Nucleinsäuremolekül(e) dadurch gekennzeichnet ist (sind), daß der Einsatz dieses(r) Nucleinsäuremoleküls(e) - zusätzlich zum Einsatz eines Nucleinsäuremoleküls (X) - in einem Verfahren zum Nachweis von Bakterien der Gattung *Salmonella* den Nachweis auch von solchen *Salmonella*-Stämmen oder *Salmonella*-Isolaten ermöglicht, deren Nachweis durch Einsatz des Nucleinsäuremoleküls (X) nicht bzw. nur mit geringerer Sensitivität gelingt.

Der erfindungsgemäße Satz von Nucleinsäuremolekülen kann dadurch gekennzeichnet sein, daß die Nucleinsäure-Isolate phylogenetisch konservierte Basensequenzen oder Bereiche dieser Basensequenzen umfassen oder darstellen. Für den Begriff der phylogenetisch konservierten Basensequenz vgl. beispielsweise WO 95/00664 oder Herder's Lexikon der Biochemie und Molekularbiologie, Ergänzungsstand 1995, Seite 132, Spektrum, Herstellung etc..

Der erfindungsgemäße Satz von Nucleinsäuremolekülen kann dadurch gekennzeichnet sein, daß die einzelnen Nucleinsäuremoleküle oder daß einzelne der Nucleinsäuremoleküle
(i) an verschiedenen phylogenetisch konservierten Basensequenzen oder
(ii) an ein und derselben phylogenetisch konservierten Basensequenz an sich nicht überlappenden Sequenzbereichen oder
(iii) an ein und derselben phylogenetisch konservierten Basensequenz an sich überlappenden Sequenzbereichen hybridisieren.

Der erfindungsgemäße Satz von Nucleinsäuremolekülen oder ein erfindungsgemäßer Satz von Nucleinsäuremolekülen, mit dem sich bei einem Verfahren zum Nachweis von Vertretern von *Salmonella enterica* subsp. *enterica, salamae, arizonae, diarizonae, houtenae, bongori* und *indica* sämtliche Vertreter dieser Subspezies nachweisen lassen, kann dadurch gekennzeichnet sein, daß der Satz für ein einzelnes, für mehrere seiner einzelnen oder für jedes seiner einzelnen Nucleinsäuremoleküle jeweils mindestens ein weiteres Nucleinsäuremolekül umfaßt, das in einem Bereich von mindestens 10 aufeinanderfolgenden Nucleotiden ihrer Nucleotidkette in weniger als 100 %, aber zumindest in 80 % der Basensequenz übereinstimmt.

Ein derartiger erfindungsgemäßer Satz von Nucleinsäuremolekülen kann dadurch gekennzeichnet sein, daß der Satz für ein einzelnes, für mehrere seiner einzelnen oder für jedes seiner einzelnen Nucleinsäuremoleküle jeweils mindestens ein weiteres Nucleinsäuremolekül umfaßt, das in einem Bereich von mindestens 10 aufeinanderfolgenden Nucleotiden ihrer Nucleotidkette in genau einer Basenposition vom anderen bzw. weiteren Nucleinsäuremolekül abweicht.

Ein erfindungsgemäßer Satz von Nucleinsäuremolekülen kann dadurch gekennzeichnet sein, daß er ein oder mehrere, jedoch nicht ausschließlich solche Nulcleinsäuremoleküle umfaßt, bei denen es sich um Fragmente der SEQ ID NO 1 gemäß WO 95/00664 oder der hierzu komplementären Sequenz handelt.

Ferner kann ein erfindungsgemäßer Satz von Nucleinsäuremolekülen dadurch gekennzeichnet sein, daß die einzelnen Nucleinsäuremoleküle an denselben Strang von Nucleinsäure-Isolaten von Vertretern von *Salmonella enterica-*Subspezies hybridisieren, die dem Verfahren zu ihrem Nachweis unterworfen werden.

Außerdem wird die der Erfindung zugrundeliegende Aufgabe durch ein Nucleinsäuremolekül gelöst, das zu einem erfindungsgemäßen Satz von Nucleinsäuremolekülen gehört oder für einen derartigen Satz verwendbar ist, wobei das Nucleinsäuremolekül dadurch gekennzeichnet ist, daß die Sequenz des Nucleinsäuremoleküls in einem Bereich von mindestens 10 aufeinanderfolgenden Nucleotiden seiner Nucleotidkette exakt mit einem Sequenzbereich mindestens eines Vertreters der genannten Salmonella *enterica*-Subspezies übereinstimmt, wobei der Sequenzbereich eine phylogenetisch konservierte Basensequenz oder einen Bereich dieser Basensequenz umfaßt oder darstellt.

Ein derartiges erfindungsgemäßes Nucleinsäuremolekül kann dadurch gekennzeichnet sein, daß es in einem Bereich von mindestens 10 aufeinanderfolgenden Nucleotiden seiner Nucleotidkette zu 100 % oder zu mindestens 80 % identisch ist zu einer entsprechenden Zahl aufeinanderfolgender Nucleotide einer oder mehrerer der folgenden Sequenzen oder ihrer komplementären Sequenzen:

| | |
|---|---|
| SEQ ID NO: 1 | ATGGATCAGAATACGCCCCG |
| SEQ ID NO: 2 | ATGGATCAGAATACACCCCG |
| SEQ ID NO: 3 | CAGAATACGCCCCGTTCGGC |
| SEQ ID NO: 4 | CAGAATACACCCCGTTCGGC |
| SEQ ID NO: 5 | CAGAATACGCCCCGTTCAGC |
| SEQ ID NO: 6 | CAACCTAACTTCTGCGCCAG |
| SEQ ID NO: 7 | CAACCTAACTTCTGCACCAG |
| SEQ ID NO: 8 | CAACCTAACCTCTGCGCCAG |
| SEQ ID NO: 9 | CAACCTAACTTCTGCGCCAG |

Ferner wird die der Erfindung zugrundeliegende Aufgabe durch ein Nucleinsäuremolekül gelöst, das dadurch gekennzeichnet ist, daß es hinsichtlich seiner Sequenz zu einem erfindungsgemäßen und vorstehend gekennzeichneten Nucleinsäuremolekül homolog ist und bei mindestens 10 aufeinanderfolgenden Nucleotiden seiner Nucleotidkette
(i) mit einem erfindungsgemäßen vorstehend gekennzeichneten Nucleinsäuremolekül identisch ist oder
(ii) in nicht mehr als einem Nucleotid von einem erfindungsgemäßen vorstehend gekennzeichneten Nucleinsäuremolekül abweicht oder
(iii) in nicht mehr als zwei Nucleotiden von einem erfindungsgemäßen vorstehend gekennzeichneten Nucleinsäuremolekül abweicht.

Ein erfindungsgemäßes Nucleinsäuremolekül kann dadurch gekennzeichnet sein, daß es 10 bis 250 und vorzugsweise 15 bis 30 Nucleotide lang ist.

Ferner kann ein erfindungsgemäßes Nucleinsäuremolekül dadurch gekennzeichnet sein, daß es einzelsträngig ist oder einen komplementären Strang aufweist.

Ferner kann ein erfindungsgemäßes Nucleinsäuremolekül dadurch gekennzeichnet sein, daß es
(i) als DNA oder
(ii) als (i) entsprechende RNA oder
(iii) als PNA vorliegt, wobei das Nucleinsäuremolekül gegebenenfalls in einer für analytische Nachweisverfahren, insbesondere auf Basis von Hybridisierung und/oder Amplifizierung, in an sich bekannter Weise modifiziert oder markiert ist.

Ferner kann ein erfindungsgemäßes Nucleinsäuremolekül dadurch gekennzeichnet sein, daß es sich um ein modifiziertes oder markiertes Nucleinsäuremolekül handelt, bei dem bis zu 20 % der Nucleotide von mindestens 10 aufeinanderfolgenden Nucleotiden seiner Nucleotidkette Bausteine darstellen, die für Sonden und/oder Primer an sich bekannt sind, insbesondere Nucleotide darstellen, die bei Bakterien nicht natürlich vorkommen.

Ferner kann ein erfindungsgemäßes Nucleinsäuremolekül dadurch gekennzeichnet sein, daß es sich um ein modifiziertes oder markiertes oder zusätzlich modifiziertes oder markiertes Nucleinsäuremolekül handelt, das in einer für analytische Nachweisverfahren an sich bekannten Weise eine oder mehrere radioaktive Gruppen, farbige Gruppen, fluoreszierende Gruppen, Gruppen zur Immobilisierung an fester Phase, Gruppen für eine indirekte oder direkte Reaktion, insbesondere für eine enzymatische Reaktion, vorzugsweise mit Hilfe von Antikörpern, Antigenen, Enzymen und/oder Substanzen mit Affinität zu Enzymen oder Enzymkomplexen, und/oder anderweitige an sich bekannte modifizierende oder modifizierte Gruppen nucleinsäureähnlichen Aufbaus aufweist.

Außerdem wird die der Erfindung zugrundeliegende Aufgabe durch einen Kit für analytische Nachweisverfahren gelöst, insbesondere zum Nachweis von Bakterien der Gattung *Salmonella,* wobei dieser Kit gekennzeichnet ist durch
(i) einen Satz von erfindungsgemäßen Nucleinsäuremolekülen oder
(ii) durch ein oder mehrere erfindungsgemäße Nucleinsäuremoleküle.

Ein erfindungsgemäßer Kit kann also einen erfindungsgemäßen Satz von Nucleinsäuremolekülen oder ein oder mehrere erfindungsgemäße Nucleinsäuremoleküle umfassen, wobei daneben die für Nucleinsäurehybridisierungen oder Nucleinsäureamplifizierungen üblichen sonstigen Komponenten vorgesehen sind, beispielsweise eine Polymerase, eine Reverstranscriptase, eine Ligase oder eine RNA-Polymerase; vgl. beispielsweise WO 95/00664.

Vorzugsweise wird ein Satz von Nucleinsäuremolekülen des erfindungsgemäßen Kits synthetisch, in zumindest zwei voneinander getrennten Syntheseansätzen hergestellt.
Der erfindungsgemäße Kit umfaßt vorzugsweise keine degenerierten Nucleinsäuremoleküle.

Schließlich wird die der Erfindung zugrundeliegende Aufgabe durch die Verwendung eines Satzes von erfindungsgemäßen Nucleinsäuremolekülen oder eines erfindungsgemäßen Kits zum Nachweis der An- oder Abwesenheit von Bakterien gelöst, welche einer Gruppe von Bakterien der Gattung *Salmonella* angehören, insbesondere von Vertretern der vorstehend genannten *Salmonalla enterica*-Subspezies.

Für die erfindungsgemäße Verwendung kann man eine Nucleinsäurehybridisierung und/oder eine Nucleinamplifizierung durchführen.

Als Nucleinsäureamplifikation kann man eine Polymerase-Kettenreaktion (PCR) durchführen.

Für die erfindungsgemäße Verwendung kann man für die nachzuweisenden Bakterien und die nicht-nachzuweisenden Bakterien Unterschiede ihrer genomischen DNA und/oder RNA an mindestens einer Nucleotidposition im Bereich eines erfindungsgemäßen Nucleinsäuremoleküls ermitteln und Vertreter einer Gruppe von Bakterien der Gattung *Salmonella* nachweisen, insbesondere von Vertretern der genannten *Salmonella enterica-*Subspezies.

Zur Detektion von Salmonellen mittels NucleinsäureHybridisierung oder -Amplifikation werden *Salmonella-*spezifische Oligonucleotide eingesetzt. *Salmonella*-spezifische Oligonukleotide sind Nucleinsäuremoleküle, 10 bis 250 Basen (vorzugsweise 15 bis 30 Basen) lang, deren Basensequenz charakteristisch für Salmonellen ist: Bei Einsatz dieser Oligonukleotide als Primer oder Sonden erfolgt - unter geeigneten Reaktionsbedingungen - eine Hybridisierung/Amplifikation nur dann, wenn DNA der nachzuweisenden Salmonellen in der Untersuchungsprobe vorhanden ist, nicht aber wenn DNA anderer Bakterien vorhanden ist.

Wie weiter unten beschrieben, können unter bestimmten Vorraussetzungen auch nicht-spezifische Oligonukleotide als Primer oder Sonden eingesetzt werden. Solche Oligonukleotide erlauben Hybridisierung bzw. Amplifikation nicht nur dann, wenn Salmonella-DNA in der Probe anwesend ist, sondern auch in Anwesenheit von DNA eines Bakteriums oder mehrerer Bakterien, das (die) nicht zur Gattung *Salmonella* gehört(en).

Da in hoch-konservierten Genregionen selbst unter sehr nahe verwandten Bakterien Austausche (z.B. Punktmutationen) in der DNA vorkommen können, sind für die Auswahl geeigneter Oligonukleotide umfangreiche DNA-Sequenzierungen bzw. Spezifitätstests (z.B. mittels Durchführung der PCR) notwendig. Dies gilt in gleichem Maße für nachzuweisende Bakterien (i.e. Salmonellen) wie nicht-nachzuweisende Bakterien (i.e. Bakterien, die nicht zur Gattung *Salmonella* gehören).

Zum Nachweis von Salmonellen werden Nucleinsäuren, vorzugsweise genomische DNA, zunächst aus den in einer zu untersuchenden Probe bzw. Bakterienkultur enthaltenen Zellen freigesetzt. Mittels Nucleinsäurehybridisierung kann dann unter Einsatz der erfindungsgemäßen *Salmonella*-spezifischen Oligonukleotide als Sonde der direkte Nachweis von *Salmonella*-Nucleinsäuren in der zu untersuchenden Probe erfolgen. Geeignet hierzu sind verschiedene dem Fachmann bekannte Verfahren, wie z.B. "Southern blot" oder "dot blot".

Bevorzugt ist jedoch, vor allem wegen der höheren Empfindlichkeit, der indirekte Nachweis, bei dem die gesuchten DNA/RNA-Sequenzen zunächst mittels der o.g. Verfahren zur Amplifikation von Nucleinsäuren, vorzugsweise PCR, amplifiziert werden. Die Amplifikation von DNA/RNA erfolgt durch Einsatz von *Salmonella*-spezifischen Oligonukleotiden. Dabei werden nur dann spezifische Amplifikate gebildet, wenn Salmonella-DNA/RNA in der zu untersuchenden Probe vorhanden ist. Durch eine nachgeschaltete Detektionsreaktion unter Verwendung von *Salmonella-*spezifischen Oligonukleotiden als Sonden kann die Spezifität des Nachweisverfahrens erhöht werden. Ebenso ist die Verwendung von nicht-spezifischen Oligonukleotiden als Sonden möglich.

Alternativ kann die Amplifikation auch in Anwesenheit eines oder mehrerer nicht-spezifischer Oligonucleotide durchgeführt werden, so daß möglicherweise auch DNA/RNA anderer, nichtnachzuweisender Mikroorganismen amplifiziert werden kann. Ein derartiges Amplifikationsverfahren ist in der Regel weniger spezifisch und sollte daher durch eine nachfolgende Detektionsreaktion mit *Salmonella*-spezifischen Oligonucleotiden als Sonde abgesichert werden.

Dem Fachmann sind verschiedene Verfahren bekannt, mit denen die bei den indirekten Verfahren entstehenden Amplifikationsprodukte nachgewiesen werden können. Dazu gehören u.A. die Visualisierung mittels Gelelektrophorese, die Hybridisierung von Sonden an immobilisierte Reaktionsprodukte [gekoppelt an Nylon- oder Nitrocellulose-Filter ("Southern blots") oder z.B. an "beads" oder Mikrotiterplatten] und die Hybridisierung der Reaktionsprodukte an immobilisierte Sonden (z.B. "reverse dot blots" oder mit Sonden gekoppelte "beads" oder Mikrotiterplatten).

Es sind eine Vielzahl verschiedener Varianten beschrieben, mit denen die beschriebenen *Salmonella*-spezifischen bzw. nicht-spezifischen Oligonukleotide zum Einsatz als Sonden und/oder Primer in direkten oder indirekten Nachweisverfahren markiert bzw. modifiziert werden können. So können diese beispielsweise radioaktive, farbige oder fluoreszierende Gruppen enthalten oder Gruppen, die eine Immobilisierung an eine feste Phase erlauben oder anderweitig modifizierte bzw. modifizierende Gruppen wie z.B. Antikörper, Antigene, Enzyme bzw. andere Substanzen mit Affinität zu Enzymen oder Enzymkomplexen. Sonden bzw. Primer können entweder natürlich vorkommende oder synthetisch hergestellte doppelsträngige oder einzelsträngige DNA oder RNA sein bzw. modifizierte Formen von DNA oder RNA, wie z.B. PNA (bei diesen Molekülen sind die Zucker-Einheiten durch Aminosäuren oder Peptide ausgetauscht). Einzelne oder mehrere Nucleotide der Sonden oder Primer können gegen analoge Bausteine (wie z.B. in der Ziel-Nucleinsäure nicht natürlich vorkommende Nucleotide) ersetzt sein. Bei den o.g. indirekten Nachweisverfahren kann die Detektion auch über ein internmarkiertes Amplifikat geführt werden. Dies kann z.B. über den Einbau von modifizierten (z.B. von mit Digoxigenin oder Fluorescein gekoppelten) Nukleosidtriphosphaten während der Amplifikationsreaktion erfolgen.

Geeignet als erfindungsgemäße *Salmonella*-spezifische Oligonukleotide sind Nucleinsäuren, vorzugsweise 15 bis 30 Basen lang, die mindestens in einer 10 Basen langen Sequenz mit den Sequenzen 1 bis 10 oder den hierzu komplementären Sequenzen übereinstimmen. Geringere Abweichungen (1 bis 2 Basen) in dieser 10 Basen langen Sequenz sind möglich, ohne daß die jeweils angegebene Spezifität bei der Amplifikation und/oder Hybridisierung verloren geht. Dem Fachmann ist bekannt, daß im Falle solcher geringeren Abweichungen die Reaktionsbedingungen entsprechend verändert werden müssen.

Um eine vollständige Detektion aller *Salmonella*-Stämme mit Hilfe der in WO 95/00664 umrissenen DNA-Region zu ermöglichen, waren umfangreiche DNA-Sequenzanalysen erforderlich. Die Sequenz dieses DNA-Bereiches wurde von 37 ausgewählten *Salmonella*-Stämmen aller 7 Subspezies bestimmt (für die meisten Stämme die Sequenz der zwischen den Primern ST15 und ST11 liegenden DNA-Region; dies entspricht Position 1275 bis 1654 von SEQ ID NO: 1 in WO 95/00664). Mögliche experimentelle Vorgehensweisen sind dem Fachmann bekannt und sollen hier nicht im einzelnen beschrieben werden, sondern die Ergebnisse kurz zusammengefasst werden. DNA der ausgewählten *Salmonella*-Stämme wurde mittels Standardverfahren präpariert, der relevante Bereich durch PCR amplifiziert und nachfolgend sequenziert. Bei der PCR sowie der nachfolgenden Sequenzierung wurden für die meisten *Salmonella*-Stämme folgende Primer eingesetzt:

| | |
|---|---|
| ST11: | AGCCAACCATTGCTAAATTGGCGCA (siehe claim 3,WO 95/00664) |
| ST15: | GGTAGAAATTCCCAGCGGGTACTG (siehe claim 3, WO 95/00664) |

Da jedoch mit mehreren Stämmen der Subspezies IIIa, IV, V und VI mit diesem Primerpaar keine, oder nur ungenügende Amplifikation stattfand, wurden in diesen Fällen folgende Primer für die PCR und die Sequenzierung eingesetzt:

| | |
|---|---|
| ST11: | AGCCAACCATTGCTAAATTGGCGCA (siehe claim 3, WO 95/00664) |
| ST14: | TTTGCGACTATCAGGTTACCGTGG (siehe claim 3, WO 95/00664) |

Ein Vergleich der DNA-Sequenzen aller 37 *Salmonella*-Stämme ergab, daß es sich zwar insgesamt um einen konservierten DNA-Bereich handelt, daß jedoch der Grad der Konservierung auf den ersten Blick nur bedingt geeignet erschien, um *Salmonella-*spezifische Oligonukleotide abzuleiten. Selbst in den höchstkonservierten Bereichen waren bei einzelnen der sequenzierten Stämme Basenaustausche zu beobachten. Interessanterweise stellte sich heraus, daß viele der Basenaustausche nur innerhalb einer Subgruppe vorkommen und daß diese zudem innerhalb dieser Subgruppe meist konserviert sind. Dies legte die Möglichkeit nahe, mehr als zwei Primer bei der PCR einzusetzen, um eine Amplifikation auch von solchen Varianten zu ermöglichen, bei denen im Bereich der Primerbindungsstellen ein oder mehrere Basenaustausche vorhanden sind. Wie dem Fachmann bekannt, werden hierzu üblicherweise degenerierte Primer, oder aber Primer mit Desoxyinosin an den variablen Stellen, eingesetzt. Daher wurden aus dem o. g. Sequenzvergleich mehrere degenerierte Oligonukleotide abgeleitet, die sich potentiell als Primer für die Detektion aller *Salmonella enterica* Subspezies eigneten. Es zeigte sich jedoch, daß diese degenerierten Primer nur bedingt für den PCR-Nachweis geeignet sind, da sie, insbesondere bei Sequenzbereichen mit hoher Komplexität, ein vermehrtes Auftreten unspezifischer Reaktionsprodukte zu Folge haben. Da bei Auftreten solcher unspezifischen Reaktionsprodukte die Sensitivität des PCR-Nachweises in der Regel leidet, wurde eine andere Vorgehensweise erprobt. Es wurden "komplementierende" Primer bei der PCR eingesetzt. Im Gegensatz zu degenerierten Primern, bei denen alle möglichen Kombinationen der einzelnen Basenaustausche in der Primer-Mischung repräsentiert sind (Anzahl der Primer = 2^{x} x 3^{y} x 4^{z}, wobei x, y und z die Anzahl der Positionen sind, an denen zwei, drei oder vier verschiedene Basen im Bereich der Primerbindungsstelle beobachtet werden) sind bei solchen komplementierenden Primern nur die tatsächlich vorkommenden Sequenzen vorhanden. Der Vorteil gegenüber degenerierten Primern besteht in der geringeren Komplexität der erfindungsgemäßen Primer-Mischung, wodurch die Wahrscheinlichkeit des Entstehens unspezifischer Amplifikationsprodukte deutlich herabgesetzt ist. Wie sich in mehreren Experimenten herausstellte, ist dies insbesondere vorteilhaft bei einem PCR-Nachweis aus Proben mit einem hohen Gehalt von "unspezifischer" DNA (DNA, die nicht aus nachzuweisenden Bakterien stammt), da ansonsten die Sensitivität des Nachweises drastisch vermindert sein kann.

Ein großer Vorteil beim Einsatz von komplementierenden Oligonukleotiden/Primern besteht in der Möglichkeit der Optimierung von bestehenden Nachweisverfahren. So können u.U. einzelne falsch negative Ergebnisse dadurch beseitigt werden, daß Oligonukleotide mit der Sequenz der zuvor nicht erfassten Stämme zusätzlich bei der PCR und/oder der Hybridisierungsreaktion eingesetzt werden.

Der DNA-Sequenzvergleich ergab mehrere kürzere DNA-Bereiche, die für die beschriebene Strategie (Einsatz von insgesamt ≥ 3 Primern in der PCR) zur Optimierung des *Salmonella-*Nachweisverfahrens potentiell geeignet schienen.
Das nachfolgende Beispiel soll dies verdeutlichen.

### Beispiel 1: Nachweis von Salmonella-Stämmen sämtlicher 7 Subspezies mit der Polymerase-Kettenreaktion

Die folgenden 3 Abschnitte der sequenzierten DNA-Region sollen als Beispiele für die beobachteten Sequenzvariationen dienen:

### Abschnitt I (Position 1336 bis 1355 von SEQ ID NO: 1 in WO 95/00664)

| | |
|---|---|
| SEQ ID NO: 1 | ATGGATCAGAATACGCCCCG |
| SEQ ID NO: 2 | ATGGATCAGAATACACCCCG |

### Abschnitt II (Position 1342 bis 1361 von SEQ ID NO: 1 in WO 95/00664)

| | |
|---|---|
| SEQ ID NO: 3 | CAGAATACGCCCCGTTCGGC |
| SEQ ID NO: 4 | CAGAATACACCCCGTTCGGC |
| SEQ ID NO: 5 | CAGAATACGCCCCGTTCAGC |

### Abschnitt III (komplementär zu Position 1483 bis 1502 von SEQ ID NO: 1 in WO 95/00664)

| | |
|---|---|
| SEQ ID NO: 6 | CAACCTAACTTCTGCGCCAG |
| SEQ ID NO: 7 | CAACCTAACTTCTGCACCAG |
| SEQ ID NO: 8 | CAACCTAACCTCTGCGCCAG |
| SEQ ID NO: 9 | CAACCTAACTTCTGCGGCAG |
| SEQ ID NO: 10 | CAGCCTAACTTCTGCGCCAG |

Zur Überprüfung ob diese Sequenz-Abschnitte für den Nachweis sämtlicher Salmonella-Stämme der 7 Subspezies geeignet sind, wurden die Oligonukleotide Sa 1 bis 10 in folgenden Kombinationen in die PCR eingesetzt:

### Primerkombination 1:

| | |
|---|---|
| Sa1/Sa2 | (je 0,2 µM Endkonzentration) |
| Sa6/Sa7/Sa8/Sa9/Sa10 | (je 0,08 µM Endkonzentration) |

### Primerkombination 2:

| | |
|---|---|
| Sa3/Sa4/Sa5 | (je 0,13 µM Endkonzentration) |
| Sa6/Sa7/Sa8/Sa9/Sa10 | (je 0,08 µM Endkonzentration) |

Aus Reinkulturen der in Tabelle la aufgeführten Salmonellen-Stämme wurde DNA mittels Standardverfahren isoliert. Je ca. 10 bis 100 ng dieser DNA-Präparationen wurde dann in Gegenwart von Primerkombination 1 bzw. Primerkombination 2, 200 µM dNTP's (Boehringer Mannheim), 1,5 mM MgCl₂, 16 mM (NH₄)₂SO₄, 67 mM Tris/HCl (pH 8,8), 0,01 % Tween 20 und 0,03 U/µl *Taq*-Polymerase (Biomaster) in die PCR eingesetzt. Die PCR wurde in einem Perkin-Elmer 9600 Thermocycler mit dem nachfolgend aufgeführten Thermoprofil durchgeführt:
- initiale Denaturierung 95 °C 5 min
- Amplifikation (35 Zyklen) 95 °C 30 sek
   63 °C 90 sek
- finale Synthese 72 °C 5 min

Nach Beendigung der PCR-Reaktion wurden die Amplifikationsprodukte mittels Agarose-Gelelektrophorese aufgetrennt und durch Anfärbung mit Ethidiumbromid visualisiert. Das erwartete Produkt von 167 bp (Primerkombination 1) bzw. 161 bp (Primerkombination 2) Länge wurde in sämtlichen Fällen beobachtet, in denen DNA von Stämmen der Gattung *Salmonella* anwesend war (vergleiche Tabelle 1a), nicht aber bei Anwesenheit von DNA der anderen getesteten Bakterien (vergleiche Tabelle 1b). Nach Beendigung des Laufes wurde die in den Gelen enthaltene DNA mittels Standard-Methoden auf Nylon-Filter transferiert und mit dem am 5'-Ende mit Digoxygenin markierten Oligonukleotid ST14 (TTTGCGACTATCAGGTTACCGTGG (siehe claim 3, WO 95/00664) zur Überprüfung der insbesonderen basengetreuen Spezifität hybridisiert. Die Hybridisierung erfolgte in 5 x SSC , 2 % Blocking Reagenz, 0,1 % Laurylsarcosin, 0,02 % SDS und 5 pmol/ml Sonde für 4 h bei 60 °C. Gewaschen wurde in 2 x SSC, 0,1 % SDS für 2 x 15 min bei 60 °C. Die Detektion erfolgte nach Standard-Methoden mittels Anti-Digoxygenin/Alkalische Phosphatase-Konjugaten in Anwesenheit von 5-Brom-4-chlor-3-indolylphosphat und 4-Nitro-Blue Tetrazoliumchlorid (Fa. Boehringer Mannheim).

Auf den Filtern wurde nur in den Fällen eine Bande beobachtet, in denen zuvor auch eine Bande auf dem Agarose-Gel sichtbar war (siehe Tabelle la). Somit wurde sowohl mittels PCR wie auch mittels Hybridisierung die Anwesenheit der 296 getesteten *Salmonella*-Stämme aus jeder der 7 Subspezies nachgewiesen. Ein positives Signal wurde für jeden dieser Stämme sowohl mit Primerkombination 1, Primerkombination 2 sowie bei der jeweils nachfolgenden Bestätigungsreaktion durch Hybridisierung mit der Sonde ST 14 erhalten. Hingegen wurde keiner der getesteten nicht zu dieser Gattung gehörenden Bakterienstämme mit diesem System erfaßt.

**Tabelle 1a: Positive Salmonella-Stämme bei der PCR-Amplifikation mit den beiden Primerkombinationen 1 oder 2 sowie bei der nachfolgenden Hybridisierung mit dem Oligonukleotid ST 14**

| Nr. | Subspezies | Serogruppe | Serovar |
|---|---|---|---|
| | *S. enterica* subsp. *enterica* | B | Abony |
| | | | Abortusovis |
| | | | Africana |
| | | | Agona |
| | | | Agona, Lactose + |
| | | | Arechavaleta |
| | | | Brandenburg |
| | | | Bredeney |
| | | | Chester |
| | | | Coeln |
| | | | Derby O: 5 - |
| | | | Duisburg |
| | | | Duisburg, monophasisch |
| | | | Heidelberg |
| | | | Heidelberg, O5- |
| | | | I 4, 12: d: - |
| | | | I 4, 12:-:- |
| | | | I 9, 12: 1, v: - |
| | | | Indiana |
| | | | Kiambu |
| | | | Kunduchi |
| | | | Paratyphi B |
| | | | Paratyphi B H 1,2 negativ |
| | | | Paratyphi B O5 - |
| | | | Reading |
| | | | Saintpaul O5 - |
| | | | Saintpaul |
| | | | Sandiego |
| | | | Schleisheim |
| | | | Schwarzengrund |
| | | | Stanley |
| | | | Stanleyville |
| | | | Typhimurium |
| | | | Typhimurium 4 : i : 1,2 (O:5-) |
| | | | Typhimurium O : 5 - |
| | | | Typhimurium, TA 1535 |
| | | | Typhimurium, TA 1537 |
| | | | Typhimurium, TA 1538 |
| | | | Typhimurium, TA 97 |
| | | | Typhimurium, TA 98 |
| | | | Typhimurium,TA 100 |
| | | C₁ | Augustenborg |
| | | | Bareilly |
| | | | Braenderup |
| | | | Choleraesuis |
| | | | Choleraesuis var. Decatur |
| | | | Choleraesuis var. Kunzendorf |
| | | | Colindale |
| | | | Concord |
| | | | Infantis |
| | | | Isangi |
| | | | Lille |
| | | | Livingstone |
| | | | Mbandaka |
| | | | Mikawasima |
| | | | Montevideo |
| | | | Ohio |
| | | | Oranienburg |
| | | | Oslo |
| | | | Richmond (2 Isolate getestet) |
| | | | Rissen |
| | | | Singapore |
| | | | Tennessee |
| | | | Thompson (2 Isolate getestet) |
| | | | Virchow |
| | | | I 6, 7 : - : - (2 Isolate getestet) |
| | | C₂ C₃ | Albany (2 Isolate getestet) |
| | | | Altona |
| | | | Apeyeme |
| | | | Bardo |
| | | | Blockley |
| | | | Bovismorbificans |
| | | | Charlottenburg |
| | | | Cottbus |
| | | | Emek |
| | | | Ferruch |
| | | | Glostrup |
| | | | Goldcoast |
| | | | Haardt |
| | | | Hadar |
| | | | Kentucky |
| | | | Litchfield |
| | | | Manchester |
| | | | Manhatten |
| | | | Molade |
| | | | München |
| | | | Newport |
| | | | Takoradi |
| | | | I 6, 8 . - . - |
| | | | I 8, 20 . - . - |
| | | D₁ | Dublin |
| | | | Durban |
| | | | Enteritidis |
| | | | Enteritidis Plasmidphage 37MD |
| | | | Enteritidis PT 4/6 |
| | | | Enteritidis, Phage |
| | | | Gallinarum |
| | | | Gallinarum-Pullorum |
| | | | Israel |
| | | | Javiana |
| | | | Kapemba |
| | | | Napoli |
| | | | Panama |
| | | | Pullorum |
| | | | I 9, 12 : - : - |
| | | D₂ | Plymoth |
| | | E₁ | Amager |
| | | | Amsterdam O : -, 15+, 34+ |
| | | | Anatum |
| | | | Anatum O 15+ |
| | | | Anatum O : 10 -, O : 15+ |
| | | | Birmingham |
| | | | Butantan |
| | | | Falkensee |
| | | | Give |
| | | | Lexington |
| | | | London |
| | | | Meleagridis |
| | | | Münster |
| | | | Münster, O : 10 -, 15+ |
| | | | Orion |
| | | | Orion O : 10 -, 15+ 34+ |
| | | | Sinstorf |
| | | | Stockholm |
| | | | Uganda (2 Isolate getestet) |
| | | | Vejle (2 Isolate getestet) |
| | | | Weltevreden |
| | | | Westhampton |
| | | | Zanzibar |
| | | | I 3, 10 : - : 6 (monophasisch) |
| | | | I 10 : - : 1,6 |
| | | E₄ | Abaetuba |
| | | | Aberdeen |
| | | | Cannstatt |
| | | | Llandoff |
| | | | Senftenberg, verzögert Lac. + |
| | | | I 1, 3, 19, : -: - |
| | | F | Chandans (2 Isolate getestet) |
| | | | Kisarawe |
| | | | Krefeld |
| | | | Liverpool |
| | | | Rubislaw |
| | | | Senftenberg |
| | | | Solt |
| | | | Telashomer |
| | | G | Grumpensis |
| | | | Havana |
| | | | Idikan |
| | | | Kedougou |
| | | | Poona |
| | | | Putten |
| | | | Worthington |
| | | | I 13, 23, : - |
| | | H | Caracas |
| | | | Charity |
| | | | Lindern |
| | | | Onderstepoort |
| | | | Sundsvall |
| | | I | Gaminara |
| | | | Hvittingfoss |
| | | | Malstatt |
| | | | Saphra |
| | | J | Bonames |
| | | K | Cerro |
| | | L | Minnesota (2 Isolate getestet) |
| | | | Ruiru |
| | | M | Cotham |
| | | | Guildford |
| | | | Ilala |
| | | | Loeben |
| | | | Mundonobo |
| | | | Nima |
| | | | Patience |
| | | | Pomona |
| | | | Taunton |
| | | | Wedding |
| | | N | Aqua |
| | | | Morningside |
| | | | Urbana |
| | | O | Adelaide |
| | | | Alachua |
| | | | Ealing |
| | | | Haga |
| | | | Monschaui |
| | | P | Lansing |
| | | | Roan (2 Isolate getestet) |
| | | | Shettfield |
| | | Q | Kokomelemle |
| | | R | Johannesburg |
| | | S | Waycross (2 Isolate getestet) |
| | | T | Waral |
| | | U | Thetford |
| | | V | Koketime (2 Isolate getestet) |
| | | | Lawra |
| | | W | Suelldorf |
| | | X | I 47, z₄, z₂₃ : - (monophasisch) |
| | | | Mountpleasant |
| | *S.* enterica subsp. *salamae* | B | II 4, 12 : a : - |
| | | C₁ | II 6, 7 : d : 1,7 |
| | | F | II 11 : g, m, s, t: z₃₉ |
| | | I | II 16 : g, m, s, t: - |
| | | J | II 17 : c : z₃₉ |
| | | | II 17 : b : e, n, x, z₁₅ |
| | | L | II 21 : z₁₀ : - |
| | | P | II 38 : d : 1,5 |
| | | R | II 1, 40 : z₄₂: 1,5,7 |
| | | S | II 41 : z₁₀, 1, 2 |
| | | T | II 42 : r : - (3 Isolate getestet) |
| | | X | II 47 : a : 1,5 (2 Isol. getestet) |
| | | | II 47 : b : 1,5 (2 Isol. getestet) |
| | | | II 47 : b : z₆ |
| | | Z | II 50 : b : z₆ (5 Isolate getestet) |
| | | O : 58 | II 58 : 1, z₁₃, z₂₈: z₆ |
| | *S. enterica* subsp. *arizonae* | J | IIIa 17 : z₄, z₃₂: - |
| | | K | IIIa 18 : z₄, z₂₃: - |
| | | P | IIIa 38 : 1, v: - |
| | | R | IIIa 40 : z₄, z₂₄: - |
| | | S | IIIa 41: z₄, z₂₃: - |
| | | U | IIIa 43 : g, z₅₁: - |
| | | V | IIIa 44 : z₄, z₃₂: - |
| | | | IIIa 44 : z₄₁, z₂₃: - |
| | | Y | IIIa 48 : (1): - |
| | | | IIIa 48 : g, z₅₁: - |
| | | | IIIa 48 : z₃₆: - |
| | | | IIIa 48 : z₄, z₂₃: - |
| | | Z | IIIa 50 : z₄, z₂₄ : - |
| | | O : 51 | IIIa 51 : z₄, z₂₃ : - |
| | | | IIIa 51 : g, z₅₁: - |
| | | O : 53 | IIIa 53 : z₄, z₂₃, z₃₂ : |
| | | | IIIa 53 : z₂₉ : - |
| | | O : 62 | IIIa 62 : z₃₆: - |
| | | O : 63 | IIIa 63 : g, z₅₁ : - |
| | *S. enterica* subsp. *diarizonae* | D₁ | IIIb 1, 9, 12 : y : Z₃₉ |
| | | I | IIIb 16 : k : - |
| | | J | IIIb 17 : z₁₀, e, n, x, z₁₅ |
| | | O | IIIb 35 : k: e, n, z₁₅ |
| | | P | IIIb 38 : 1, v: z₅₃ |
| | | | IIIb 38 : 1, v: z₅₄ |
| | | T | IIIb 42 : k : z₃₅ |
| | | X | IIIb 47 : b : z₆ |
| | | | IIIb 47 : k : z₃₅ |
| | | | IIIb 47 : r: z₅₃ |
| | | | IIIb 47 : - : - |
| | | Y | IIIb 48 : (k) : z₅₃ |
| | | Z | IIIb 50 : k : z |
| | | | IIIb 50 : r : z |
| | | O : 53 | IIIb 53 : l, k : z |
| | | O : 60 | IIIb 60 : z₅₂ : z₅₃ |
| | | O : 61 | IIIb 61 : i : z |
| | | | IIIb 61 : 1, v : 1,5,7 |
| | | | IIIb 61 : 1, v : 1,5,7: (z₅₇) |
| | | | IIIb 61 : r : z₅₃ |
| | *S. enterica* subsp. *houtenae* | F | IV 11 : z₄, Z₂₃: - |
| | | I | IV 16 : z₄, z₃₂: - |
| | | I | IV 16 : z₄, z₃₂: - |
| | | J | IV 17 : z₂₉: - |
| | | K | IV 18 : z₃₆, z₃₈ : - |
| | | L | IV 21: g, z₅₁: - |
| | | U | IV 43 : z₄, z₂₃: - |
| | | | IV 43 : z₄, z₃₂: - |
| | | V | IV 44 : z₄, z₃₂: - |
| | | Y | IV 48 : z₂₉: - |
| | | Z | IV 50 : z₄, z₂₃: - |
| | *S. enterica* subsp. *bongori* | R | V 40 : z₃₅: - |
| | | | V 40 : z₈₁: - |
| | | V | V 44 : d: - |
| | | | V 44 : z₃₉: - |
| | | Y | V 48 : z₃₅: - |
| | *S. enterica* subsp. *indica* | S | VI 41 : b: 1,7 |
| | | W | VI 45 : a: e,n,x, (z₁₇) |
| | | Y | VI 48 : z₁₀ : 1,5 |
| | | | VI 48 : z₄₁ : - |
| | | | VI 1, V : z₆₇ |

Bezugsquelle: bgVV Berlin, (Robert von Ostertag Institut, Marienfelde) und Institut für Mikrobiologie und Hygiene, Hamburg (Prof. Aleksic). Bei den Stämmen mit der Bezeichnung TA handelt es sich um Stämme, die für den Ames Test eingesetzt werden (Maron, D.M. & Ames, B.N., Mutation Research 113, 173-215 (1983).

**Tabelle 1b: Negative Stämme von Nicht-Salmonella Spezies bei der PCR-Amplifikation mit den beiden Primerkombinationen 1 oder 2 sowie der nachfolgenden Hybridisierung mit dem Oligonukleotid ST 14**

| Nr. | Spezies | Herkunft |
|---|---|---|
| | *Bacillus subtilis* | ATCC 6051 |
| | *Citrobacter freundii* | DSM 30040 |
| | *Clostridium bifermentans* | DSM 630 |
| | *Enterobacter agglomerans* | IfGB 0202 |
| | *Enterobacter cloacae* | DSM 30054 |
| | *Erwinia carotovora* | DSM 30168 |
| | *Escherichia coli* | ATCC 8739 |
| | *Hafnia alvei* | IfGB 0101 |
| | *Klebsiella oxytoca* | DSM 5175 |
| | *Klebsiella pneumoniae* | ATCC 13883 |
| | *Klebsiella oxytoca* | DSM 5175 |
| | *Lactobacillus spec.* | ATCC 20182 |
| | *Listeria monocytogenes* | ATCC 19118 |
| | *Pediococcus domnatus* | IfGB 0101 |
| | *Proteus vulgaris* | DSM 2041 |
| | *Pseudomonsas fluorescens* | DSM 6290 |
| | *Serratia marcescens* | IfGB 0101 |
| | *Shigella flexneri* | DSM 4782 |
| | *Staphylococcus aureus* | ATCC 6538 |
| | *Yersinia enterocolitica* | DSM 4780 |

## Patentansprüche

1. Nucleinsäuremolekül zum Nachweis von Vertretern von *Salmonella enterica*-Subspezies *enterica, salamae, arizonae, diarizonae, houtenae, bongori* und *indica*, mit einer oder mehreren der folgenden Sequenzen oder ihren komplementären Sequenzen:
| | |
|---|---|
| SEQ ID NO: 1 | ATGGATCAGAATACGCCCCG |
| SEQ ID NO: 2 | ATGGATCAGAATACACCCCG |
| SEQ ID NO: 3 | CAGAATACGCCCCGTTCGGC |
| SEQ ID NO: 4 | CAGAATACACCCCGTTCGGC |
| SEQ ID NO: 5 | CAGAATACGCCCCGTTCAGC |
| SEQ ID NO: 6 | CAACCTAACTTCTGCGCCAG |
| SEQ ID NO: 7 | CAACCTAACTTCTGCACCAG |
| SEQ ID NO: 8 | CAACCTAACCTCTGCGCCAG |
| SEQ ID NO: 9 | CAACCTAACTTCTGCGGCAG |
| SEQ ID NO: 10 | CAGCCTAACTTCTGCGCCAG |
wobei das Nucleinsäuremolekül
(i) in nicht mehr als einem Nucleotid von einem Nucleinsäuremolekül der SEQ ID NO:1 bis SEQ ID NO:10 abweicht, oder
(ii) in nicht mehr als zwei Nucleotiden von einem Nucleinsäuremolekül der SEQ ID NO:1 bis SEQ ID NO:10 abweicht.

2. Nucleinsäuremolekül nach Anspruch 1, **dadurch gekennzeichnet, dass** es an denselben Strang von Nucleinsäure-Isolaten von Vertretern von *Salmonella enterica*-Subspezies hybridisiert, die dem Verfahren zu ihrem Nachweis unterworfen sind.

3. Nucleinsäuremolekül nach einem der vorhergehenden Ansprüche, **dadurch gekennzeichnet, dass** es exakt mit einem Sequenzbereich mindestens eines Vertreters von *Salmonella enterica*-Subspezies übereinstimmt, wobei der Sequenzbereich eine phylogenetisch konservierte Basensequenz umfasst.

4. Nucleinsäuremolekül nach einem der vorhergehenden Ansprüche, **dadurch gekennzeichnet, dass** es einzelsträngig ist oder einen komplementären Strang aufweist.

5. Nucleinsäuremolekül nach einem der vorhergehenden Ansprüche, **dadurch gekennzeichnet, dass** es
(i) als DNA, oder
(ii) als (i) entsprechende RNA, oder
(iii) als PNA vorliegt,
wobei das Nucleinsäuremolekül gegebenenfalls für analytische Nachweisverfahren, insbesondere auf Basis von Hybridisierung und/oder Amplifizierung modifiziert oder markiert ist.

6. Nucleinsäuremolekül nach Anspruch 5, **dadurch gekennzeichnet, dass** es eine oder mehrere radioaktive Gruppen, farbige Gruppen, fluoreszierende Gruppen, Gruppen zur Immobilisierung an fester Phase, Gruppen für eine indirekte oder direkte Reaktion, insbesondere für eine enzymatische Reaktion, vorzugsweise mit Hilfe von Antikörpern, Antigenen, Enzymen und/oder Substanzen mit Affinität zu Enzymen oder Enzymkomplexen, aufweist.

7. Primerkombination zum Nachweis von Vertretern von *Salmonella enterica*-Subspezies *enterica, salamae, arizonae, diarizonae, houtenae, bongori* und *indica* mittels Polymerase-KettenReaktion, bestehend aus Primerkombination 1, die SEQ ID NO: 1 und SEQ ID NO:2 sowie SEQ ID NO:6, SEQ ID NO:7, SEQ ID NO:8, SEQ ID NO:9 und SEQ ID NO:10 enthält, oder Primerkombination 2, die SEQ ID NO: 3, SEQ ID NO: 4 und SEQ ID NO:5 sowie SEQ ID NO:6, SEQ ID NO:7, SEQ ID NO:8, SEQ ID NO:9 und SEQ ID NO:10 enthält.

8. Kit für analytische Nachweisverfahren, zum Nachweis von Bakterien der Gattung *Salmonella*, **gekennzeichnet durch**
(i) ein Nucleinsäuremolekül nach einem der Ansprüche 1 bis 6; oder
(ii) eine Primerkombination nach Anspruch 7.

9. Kit nach Anspruch 8, **dadurch gekennzeichnet, dass** die Nucleinsäuremoleküle synthetisch in mindestens zwei voneinander getrennten Syntheseansätzen hergestellt wurden.

10. Kit nach Anspruch 9, **dadurch gekennzeichnet, dass** der Kit keine degenerierten Nucleinsäuremoleküle umfasst.

11. Verwendung eines Nucleinsäuremoleküls nach einem der Ansprüche 1 bis 6, einer Primerkombination nach Anspruch 7 oder eines Kits nach einem der Ansprüche 8 bis 10, zum Nachweis der An- oder Abwesenheit von Bakterien, welche Vertretern von *Salmonella enterica*-Subspezies angehören.

12. Verwendung nach Anspruch 11, **dadurch gekennzeichnet, dass** man eine Nucleinsäurehybridisierung und/oder Nucleinsäureamplifizierung durchführt.

13. Verwendung nach Anspruch 12, **dadurch gekennzeichnet, dass** man eine Polymerase-Kettenreaktion (PCR) als Nucleinsäureamplifikation durchführt.

14. Verwendung nach einem der Ansprüche 11 bis 13, **dadurch gekennzeichnet, dass** man für die nachzuweisenden Bakterien Unterschiede ihrer genomischen DNA und/oder RNA an mindestens einer Nucleotidposition in einem Nucleinsäuremolekül nach einem der Ansprüche 1 bis 6 ermittelt und Vertreter der Gruppe von Bakterien der Gattung *Salmonella* nachweist, insbesondere Vertreter von *Salmonella enterica*-Subspezies.

## Claims

1. Nucleic acid molecule for the detection of representatives of *Salmonella enterica* subspecies *enterica, salamae, arizonae, diarizonae, houtenae, bongori* and *indica,* of one or more of the following sequences or their complementary sequences:
| | |
|---|---|
| SEQ ID NO: 1 | ATGGATCAGAATACGCCCCG |
| SEQ ID NO: 2 | ATGGATCAGAATACACCCCG |
| SEQ ID NO: 3 | CAGAATACGCCCCGTTCGGC |
| SEQ ID NO: 4 | CAGAATACACCCCGTTCGGC |
| SEQ ID NO: 5 | CAGAATACGCCCCGTTCAGC |
| SEQ ID NO: 6 | CAACCTAACTTCTGCGCCAG |
| SEQ ID NO: 7 | CAACCTAACTTCTGCACCAG |
| SEQ ID NO: 8 | CAACCTAACCTCTGCGCCAG |
| SEQ ID NO: 9 | CAACCTAACTTCTGCGGCAG |
| SEQ ID NO: 10 | CAGCCTAACTTCTGCGCCAG |
wherein the nucleic acid molecule
(i) differs from a nucleic acid molecule of SEQ ID NO:1 to SEQ ID NO:10 in not more than 1 nucleotide, or
(ii) differs from a nucleic acid molecule of SEQ ID NO:1 to SEQ ID NO:10 in not more than 2 nucleotides.

2. Nucleic acid molecule according to claim 1, **characterized in that** it hybridizes to the same strand of nucleic acid isolates of representatives of *Salmonella enterica* subspecies that are being subjected to the process for the detection.

3. Nucleic acid molecule according to any of the preceeding claims, **characterized in that** it corresponds exactly to a sequence region of at least one representative of *Salmonella enterica* subspecies, the sequence region comprising a phylogenetically conserved base sequence.

4. Nucleic acid molecule according to any one of the preceding claims, **characterized in that** it is single-stranded or has a complementary strand.

5. Nucleic acid molecule according to any one of the preceding claims, **characterized in that** it is present
(i) as DNA, or
(ii) as RNA corresponding to (i), or
(iii) as PNA,
the nucleic acid molecule where appropriate having been modified or labeled for analytical detection processes, especially based on hybridization and/or amplification.

6. Nucleic acid molecule according to claim 5, **characterized in that** it comprises one or more radioactive groups, colored groups, fluorescent groups, groups for immobilization on a solid phase, groups for an indirect or direct reaction, especially for an enzymatic reaction, preferably using antibodies, antigenes, enzymes and/or substances having an affinity for enzymes or enzyme complexes.

7. Primer combination for the detection of representatives of *Salmonella enterica* subspecies *enterica, salamae, arizonae, diarizonae, houtenae, bongori* and *indica* using polymerase chain reaction, consisting of primer combination 1 containing the SEQ ID NO: 1 and SEQ ID NO:2 as well as SEQ ID NO:6, SEQ ID NO:7, SEQ ID NO:8, SEQ ID NO:9 and SEQ ID NO:10, or primer combination 2 containing SEQ ID NO: 3, SEQ ID NO: 4 and SEQ ID NO:5 as well as SEQ ID NO:6, SEQ ID NO:7, SEQ ID NO:8, SEQ ID NO:9 and SEQ ID NO:10.

8. Kit for analytical detection processes, for the detection of bacteria of the *Salmonella* genus, **characterized by**
(i) a nucleic acid molecule according to any one of claims 1 to 6; or
(ii) a primer combination according to claim 7.

9. Kit according to claim 8, **characterized in that** the nucleic acid molecules were produced synthetically in at least two separate synthesis batches.

10. Kit according to claim 9, **characterized in that** the kit does not comprise any degenerate nucleic acid molecules.

11. Use of a nucleic acid molecule according to any one of claims 1 to 6, of a primer combination according to claim 7 or of a kit according to any one of claims 8 to 10 to detect the presence or absence of bacteria belonging to representatives of *Salmonella enterica*-subspecies.

12. Use according to claim 11, **characterized in that** nucleic acid hybidization and/or nucleic acid amplification is carried out.

13. Use according to claim 12, **characterized in that** a polymerase chain reaction (PCR) is carried out as nucleic acid amplification.

14. Use according to any one of claims 11 to 13, **characterized in that** differences between the genomic DNA and/or RNA of the bacteria to be detected are determined at at least one nucleotide position in a nucleic acid molecule according to any one of claims 1 to 6 and representatives of the group of bacteria of the *Salmonella* genus are detected, especially representative of *Salmonella enterica* subspecies.

## Revendications

1. Molécule d'acide nucléique pour la détection de représentants de *Salmonella enterica -* sous-espèces *enterica, salamae, arizonae, diarizonae, houtenae, bongori* et *indica*, avec une ou plusieurs des séquences suivantes ou leurs séquences complémentaires :
| | |
|---|---|
| SEQ ID N°1 | ATGGATCAGAATACGCCCCG |
| SEQ ID N°2 | ATGGATCAGAATACACCCCG |
| SEQ ID N°3 | CAGAATACGCCCCGTTCGGC |
| SEQ ID N°4 | CAGAATACACCCCGTTCGGC |
| SEQ ID N°5 | CAGAATACGCCCCGTTCAGC |
| SEQ ID N°6 | CAACCTAACTTCTGCGCCAG |
| SEQ ID N°7 | CAACCTAACTTCTGCACCAG |
| SEQ ID N°8 | CAACCTAACCTCTGCGCCAG |
| SEQ ID N°9 | CAACCTAACTTCTGCGGCAG |
| SEQ ID N°10 | CAGCCTAACTTCTGCGCCAG |
où la molécule d'acide nucléique :
(i) ne diffère pas par plus d'un nucléotide d'une molécule d'acide nucléique de SEQ ID N°1 à SEQ ID N°10, ou
(ii) ne diffère pas par plus de deux nucléotides d'une molécule d'acide nucléique de SEQ ID N°1 à SEQ ID N°10.

2. Molécule d'acide nucléique selon la revendication 1, **caractérisée en ce qu'**elle s'hybride sur le même brin d'isolats d'acide nucléique de représentants de sous-espèces de *Salmonella enterica,* qui sont soumis au procédé pour leur détection.

3. Molécule d'acide nucléique selon l'une des revendications précédentes, **caractérisée en ce qu'**elle concorde exactement avec un domaine de séquence d'un représentant de sous-espèces de *Salmonella enterica,* où le domaine de séquence comprend une séquence de bases conservée de manière phylogénétique.

4. Molécule d'acide nucléique selon l'une des revendications précédentes, **caractérisée en ce qu'**elle est un brin unique ou elle présente un brin complémentaire.

5. Molécule d'acide nucléique selon l'une des revendications précédentes, **caractérisée en ce qu'**elle se présente
(i) comme un ADN, ou
(ii) comme un ARN correspondant à (i), ou
(iii) comme un PNA,
où la molécule d'acide nucléique est le cas échéant modifiée ou marquée pour le procédé de détection analytique, en particulier sur base d'une hybridation et/ou d'une amplification.

6. Molécule d'acide nucléique selon la revendication 5, **caractérisée en ce qu'**elle présente un ou plusieurs groupes radioactifs, groupes colorés, groupes fluorescents, groupes pour l'immobilisation sur une phase solide, groupes pour une réaction indirecte ou directe, en particulier pour une réaction enzymatique, de préférence à l'aide d'anticorps, antigènes, enzymes et/ou substances avec affinité pour des enzymes ou des complexes enzymatiques.

7. Combinaison d'amorces pour la détection de représentants de *Salmonella enterica -* sous-espèces *enterica, salamae, arizonae, diarizonae, houtenae, bongori* et *indica,* par une réaction en chaîne de la polymérase, consistant en la combinaison d'amorces 1, qui contient SEQ ID N°1 et SEQ ID N°2, ainsi que SEQ ID N°6, SEQ ID N°7, SEQ ID N°8, SEQ ID N°9 et SEQ ID N°10, ou en la combinaison d'amorces 2, qui contient SEQ ID N°3 et SEQ ID N°4 et SEQ ID N°5, ainsi que SEQ ID N°6, SEQ ID N°7, SEQ ID N°8, SEQ ID N°9 et SEQ ID N°10.

8. Kit pour un procédé de détection analytique, pour la détection de bactéries du genre *Salmonella,* **caractérisé par** :
(i) une molécule d'acide nucléique selon une des revendications 1 à 6, ou
(ii) une combinaison d'amorces selon la revendication 7.

9. Kit selon la revendication 8, **caractérisé en ce que** les molécules d'acide nucléique sont préparées de manière synthétique, en au moins deux charges de synthèse séparées.

10. Kit selon la revendication 9, **caractérisé en ce que** le kit ne comprend pas de molécules d'acide nucléique dégénérées.

11. Utilisation d'une molécule d'acide nucléique selon une des revendications 1 à 6, d'une combinaison d'amorces selon la revendication 7 ou d'un kit selon une des revendications 8 à 10, pour la détection de la présence ou de l'absence de bactéries, qui sont des représentants de sous-espèces de *Salmonella enterica.*

12. Utilisation selon la revendication 11, **caractérisée en ce** l'on réalise une hybridation d'acide nucléique et/ou une amplification d'acide nucléique.

13. Utilisation selon la revendication 12, **caractérisée en ce** l'on réalise une réaction en chaîne de la polymérase (PCR) comme amplification d'acide nucléique.

14. Utilisation selon l'une des revendications 11 à 13, **caractérisée en ce** l'on détermine pour les bactéries à détecter, des différences de leur ADN génomique et/ou ARN en au moins une position de nucléotide dans une molécule d'acide nucléique selon une des revendications 1 à 6, et on détecte des représentants du groupe de bactéries du genre *Salmonella,* en particulier des représentants de sous-espèces de *Salmonella enterica.*
